# EUROPÄISCHE PATENTSCHRIFT

(11) **EP 1 903 003 B1**
(45) Veröffentlichungstag und Bekanntmachung des Hinweises auf die Patenterteilung: **05.07.2017**
(21) Anmeldenummer: 06013915.1
(22) Anmeldetag: 05.07.2006
(51) Int. Cl.: C01B 21/24, B01J 19/12, C01B 21/28

(54) **Verfahren zur photolytischen Erzeugung von Stickstoffmonoxid**
Method for photolytic production of nitric oxide
Procede de production photolytique de monoxyde d'azote

(43) Veröffentlichungstag der Anmeldung: 26.03.2008
(60) Teilanmeldung: 15197731.1
(73) Patentinhaber: BSN medical GmbH, 20253 Hamburg (DE)
(72) Erfinder: Suschek, Christoph, 40764 Langenfeld (DE)
(74) Vertreter: Jostarndt Patentanwalts-AG

(56) Entgegenhaltungen:
- EP-B1- 0 560 928
- WO-A-94/20415
- US-A- 3 840 342
- US-A- 5 094 815
- US-A- 5 374 710

## Beschreibung

Die vorliegende Erfindung betrifft ein Verfahren zur photolytischen Erzeugung von Stickstoffmonoxid. Stickstoffmonoxid (NO) ist ein biologisch wichtiges Molekül für die inner- und interzelluläre Reizleitung und für immunologische Reaktionen, welches normalerweise auch im menschlichen Körper gebildet wird. Die Entspannung von Blutgefässen wird von NO ebenso beeinflusst wie die Entspannung der Alveolen oder der glatten Muskulatur im gastrischen Bereich. Ein Mangel an NO kann umgekehrt dazu führen, dass die Relaxation der glatten Muskulatur unterbleibt und damit Konstriktionen auftreten. Eine solche Konstriktion äußert sich beispielsweise im bronchialen Bereich in Form von Asthma. Viele dieser Symptome lassen sich bekanntermaßen durch Inhalation NO-haltiger Gasgemische mit Konzentrationen im Bereich von 0,5 ppm bis 200 ppm beheben. Bei Neugeborenen mit Atemproblemen, beispielsweise im Falle eines Lungenhochdrucks, wenn das Kind einen Blausuchtsanfall hat, wird Stickstoffmonoxid als Medikament eingesetzt. Das Besondere daran ist, das NO so schnell wirkt wie kein anderes Medikament. Auch eine Herz-Lungen-Maschine kann nicht so schnell angeschlossen werden. Die Verwendung von Stickstoffmonoxid im pharmazeutischen Bereich ist beispielsweise in der EP 0 560 928 beschrieben.

Bisher wird ein für therapeutische Zwecke genutztes, NO-haltiges Inhalationsgas aus Gasflaschen (Industriegas) bereitgestellt, deren Lagerung und Handhabung in einer Klinik oder einer anderen Therapieeinrichtung aufgrund der erforderlichen Sicherheitsmaßnahmen aufwendig ist. Dies gilt insbesondere für eine mobile Vorrichtung. Zudem muss die Qualität des gelagerten Gases für medizinische Anwendungen hohen Anforderungen genügen, die den Aufwand für Herstellung und Lagerung weiter erhöhen. Schon eine geringe Verunreinigung des Gases führt nämlich zur Bildung unerwünschter und eventuell giftiger Nebenprodukte. Die europäischen Arzneimittel- und Gesundheitsbehörden haben demgemäß hohe Anforderungen an die Reinheit des zu verwendenden Stickstoffmonoxids gestellt.

Neben der Verwendung von "technischen" NO-Gasen für die medizinische Anwendung, gibt es Verfahren zur plasmachemischen Erzeugung von Stickstoffmonoxid. Aus den Druckschriften WO 95/07610 A, US 5,396,882 A und DE 198 23 748 A sind Verfahren zur plasmachemischen Erzeugung von NO bekannt, bei dem NO unter Einwirkung einer Glimm-, Funken- oder Lichtbogenentladung in einem Stickstoff (N₂) und Sauerstoff (O₂) enthaltenden Betriebsgas erzeugt wird. Eine Gasentladung der beschriebenen Art führt, wenn sie bei zu niedrigen Temperaturen durchgeführt wird (wie bei der Glimmentladung zu beobachten), zu einer geringen Effizienz der NO-Erzeugung in einem Gasgemisch. Zudem wird unter diesen Bedingungen bevorzugt das für Inhalationszwecke unerwünschte NO₂-Radikal (NO₂^{•}) erzeugt. Um das NO₂⁻Radikal NO₂^{•} aus dem Inhalationsgas zu entfernen, ist der Einsatz einer aufwendigen Absorbertechnik erforderlich. Der Nachteil eines Absorbers liegt insbesondere darin, dass das Absorbermaterial häufig ausgetauscht oder wiederaufbereitet werden muss. Die im Vergleich zu einer Glimmentladung energiereichere Funken- oder Lichtbogenentladung bewirkt eine vergleichsweise starke Gasaufheizung, wodurch eine entsprechend effiziente NO-Erzeugung erzielt wird. Die insbesondere am Ansatzpunkt des Funkens hohe thermische Belastung der Elektroden führt jedoch in nachteiliger Weise zu einer starken Elektrodenerosion, d. h. einer fortschreitenden Zersetzung des Elektrodenmaterials. Aufgrund dieser Elektrodenerosion ist das Verfahren einerseits wartungsintensiv, da die Elektroden sehr verschleißanfällig sind. Anderseits muss verhindert werden, dass das im Inhalationsgas fein verteilte erodierte Elektrodenmaterial in die Atmungswege eines Patienten gelangt. Dies erfordert eine aufwendige Reinigung des Inhalationsgases.

Ferner ist auch eine Generierung von NO durch Photolyse möglich. Danach werden z.B. die in einer nitrithaltigen Lösung (z.B. Natriumnitrit) enthaltenen Nitrit-Ionen (NO₂⁻) mittels elektromagnetischer Strahlung (z.B. UVA-Strahlung mit Wellenlängen zwischen 320 und 440 nm) gespalten (Photolyse), wodurch NO generiert wird. Unter reduktiven Bedingungen bzw. unter Schutzgas (z.B. Stickstoff) verläuft der durch elektromagnetische Strahlung induzierte Zerfall von Nitrit über unterschiedliche, zum Teil auch parallele, thermodynamisch jedoch verschieden gewichtete Kanäle. Es kann angenommen werden, dass im Kanal 1 (Reaktionen 1 bis 5) UVA-Strahlung (mit einem Optimum bei 354 bis 366 nm) Nitrit zum Stickstoffmonoxidradikal (NO^{•}) und zum Sauerstoffradikalanion (O^{•-}) spaltet (Gleichung 1). Das zuletzt genannte Produkt initiiert im Folgenden in wässrigen Lösungen die Bildung des reaktiven Hydroxylradikals (OH^{•}) (Gleichung 2). Das Hydroxylradikal reagiert mit Nitrit, was zur Bildung des Nitrogendioxidradikals (NO₂^{•}) führt (Gleichung 3). Dieses kann mit Stickstoffmonoxid zu Dinitrogentrioxid (N₂O₃) weiterreagieren (Gleichung 4).

NO₂⁻+ hv → NO^{•}+O^{•-} (1)

O^{•-} + H₂O → OH^{•} + OH⁻ (2)

NO₂⁻ + OH^{•} → NO₂^{•} + OH⁻ (3)

NO₂^{•} + NO^{•} → N₂O₃ (4)

N₂O₃ + H₂O → 2 NO₂⁻ + 2 H⁺ (5)

In Kanal 2 (Gleichungen 6-10) scheinen Hydroxylradikale unter den genannten Bedingungen keine Rolle zu spielen, jedoch werden ein "aquatisiertes" Elektron (e⁻_{aq}) sowie ein Stickstoffdioxidradikal gebildet (Gleichung 6). Das Elektron wird bei einem Überschuss an Nitrit auf dieses übertragen und das daraus resultierende Nitritanion (Gleichung 7) wird in Wasser zum NO-Radikal reduziert (Gleichung 8). Die folgenden Reaktionen in Gleichungen (9) und (10) entsprechen denen in Gleichungen (4) und (5). Die Gewichtung von Kanal 1 zum Kanal 2 bildet dabei ein Verhältnis von ca. 40:60.

NO₂⁻ + hv → NO₂^{•} + e⁻_{aq} (6)

e⁻_{aq} + NO₂⁻ → NO₂^{- -} (7)

NO₂⁻⁻ + H₂O → NO^{•}+ 2OH⁻ (8)

NO^{•} + NO₂^{•} → N₂O₃ (9)

N₂O₃ + H₂O → 2 NO₂⁻ + 2 H⁺ (10)

Wie aus den Reaktionen 1 bis 10 deutlich wird, wird der photolytische Zerfall von Nitrit von einer parallelen Produktion reaktiver und zytotoxischer, chemischer Spezies begleitet. Aus den Reaktionen in Gleichungen (4) und (9) wird zudem ersichtlich, dass NO₂-Radikale (NO₂^{•}) mit dem in Gleichung (1) gebildeten NO rückreagieren können.

Der vorliegenden Erfindung liegt daher die Aufgabe zugrunde, ein verbessertes Verfahren zur Erzeugung von Stickstoffmonoxid in einem besonderen hohen Reinheitsgrad bereitzustellen, welches vorzugsweise im Wesentlichen frei von weiteren reaktiven oder toxischen, insbesondere zytotoxischen Spezies sein soll.

Gelöst wird diese Aufgabe durch ein Verfahren zur photolytischen Herstellung von Stickstoffmonoxid durch Spaltung einer Ausgangssubstanz, das gemäß dem Hauptanspruch so durchgeführt wird, dass mehrfach oxidierte Stickoxide, Sauerstoffradikalanionen oder Hydroxylradikale abgebaut oder neutralisiert werden.

Es wurde erkannt, dass durch den Einsatz von mindestens einem System, welches mehrfach oxidierte Stickoxide, Sauerstoffradikalanionen oder Hydroxylradikale abbaut oder neutralisiert, während der Generierung von Stickstoffmonoxid die Bildung der genannten reaktiven Zwischenprodukte des lichtinduzierten Nitritzerfalls (NO₂^{•}, O^{•-}, OH^{•}, e⁻_{aq}) unterbunden wird oder deren Eliminierung erfolgt, während gleichzeitig die Generierung von Stickstoffmonoxid nicht gemindert wird. Somit wird erfindungsgemäß die Ausbeute frei verfügbarem NO erhöht und die Reinheit des Gases erhöht.

Der Anstieg der NO-Freisetzung sowie der hohe Reinheitsgrad beruht auf einer reaktionsbedingten Eliminierung der reaktiven Zwischenprodukte, z.B. gemäß den folgenden Reaktionen (11) bis (17).

N₂O₃ + RS⁻ → NO₂⁻ + RSNO (11)

RSNO + hv → NO^{•} + RS^{•} (12)

NO₂^{•}+ RS⁻ → NO₂⁻ + RS^{•} (13)

NO^{•}+ RS → RSNO (14)

BA + OH^{•} → BA-OH (15)

VitC+ NO₂^{•} → NO₂⁻ + VitC^{•-} (16)

Trol+ NO₂^{•} → NO₂⁻ + Trol^{•-} (17)

(Abkürzungen: RS⁻, Thiol; GSNO, S-Nitros-Thiol; GS^{•}, Thioylradikal; BA, Benzoesäure; VitC, Vitamin C, Askorbat, Askorbinsäure; VitC^{•}, das Radikal von VitC; Trol, Trolox; Trol^{•}, das Radikal von Trolox).

Das erfindungsgemäße Verfahren ermöglicht durch die Präsenz dieser oder anderer funktionsgleicher Systeme während der Bildung von Stickstoffmonoxid eine hohe Ausbeute an Stickstoffmonoxid, während gleichzeitig die Bildung von unerwünschten (mehrfach) oxidierten Stickoxiden, insbesondere des NO₂^{•}, sowie von Hydroxylradikalen und reaktiven aquatisierten Elektronen effizient verhindert wird, oder diese Substanzen nach ihrer Entstehung eliminiert werden bzw. nur noch in so geringem Maße erzeugt werden können, dass sie in Lösung verbleiben und nicht in die Gasphase übertreten können. Somit können diese Substanzen z.B. keinen pathologisch relevanten Schaden in Folge der Inhalation der Inhalationsgase verursachen.

Als Systeme, welche reaktive mehrfach oxidierte Stickoxidspezies (z.B. Stickstoffdioxid, (NO₂)-Radikale), Sauerstoffradikalanionen oder Hydroxylradikale abbauen oder neutralisieren, werden vorzugsweise reaktive Sauerstoffspezies oder Stickoxidspezies (ROS oder RNS)-abbauende bzw. -neutralisierende Substanzen verwendet. Diese werden nachfolgend auch als Antioxidantien definiert und zusammengefasst. Weiter bevorzugt handelt es sich um Askorbinsäure, Askorbat, Vitamin E und seine Derivate, Thiole, Radikalfänger oder ROS- und RNS-abbauend Enzyme.

Erfindungsgemäß hat sich darüber hinaus herausgestellt, dass die Bindung oder Eliminierung der genannten reaktiven Zwischenprodukte des lichtinduzierten Nitritzerfalls (NO₂^{•}, O^{•-}, OH^{•}, e⁻_{aq}) auch in einem neutralen pH-Bereich erfolgen kann, wobei aus Nitrit eine maximale NO-Freisetzung in einer maximalen Reinheit des NO-Gases erreicht werden kann.

Unter sauren Bedingungen (pH<7,0) wird in wässrigen Lösungen allerdings der "spontane" Nitritzerfall begünstigt. Entsprechend der Gleichungen 18-20 befindet sich das Nitritanion (NO₂⁻) in wässrigen Lösungen in einem Gleichgewicht mit seiner konjugierten Säure, der salpetrigen Säure (HNO₂). HNO₂ seinerseits befindet sich im Gleichgewicht mit Distickstofftrioxid (N₂O₃), welches spontan zu NO^{•} and NO₂^{•} zerfällt.

NO₂⁻ + H⁺ ⇆ HNO₂ (18)

2 HNO₂ ⇆ N₂O₃ + H₂O (19)

N₂O₃ ⇆ NO^{•} + NO₂^{•} (20)

In einer Ausführungsform des erfindungsgemäßen Verfahrens erfolgt daher die UVAinduzierte Generierung von Stickstoffmonoxid vorzugsweise in einem pH-Intervall von 0 bis 12, insbesondere von 1 bis 10, besonders bevorzugt von 1,5 bis 6, speziell von 2 bis 6, ganz speziell von 2,5 bis 4.

Je nach eingesetzter Nitrit- oder Antioxidantkonzentration sowie je nach Höhe des eingesetzten physikalischen Dekompensationsreizes, der zum Zerfall von Nitrit führt, kann durch das erfindungsgemäße Verfahren eine hohe Konzentration an Stickstoffmonoxid im Trägergas erreicht werden. So ist es durch das erfindungsgemäße Verfahren möglich, ausgehend von Nitrit, einen Stickstoffmonoxidhaltigen Trägergasstrom zu erzeugen, welcher vorzugsweise eine Konzentration an Stickstoffmonoxid von 1000 ppm oder höher aufweist. Dieser hochkonzentrierte Trägergasstrom übersteigt die für medizinische Anwendungen üblicherweise verträgliche NO-Konzentration von 5 bis zu 200 ppm um ein Mehrfaches.

Die Menge des generierten Stickstoffmonoxids in dem erfindungsgemäßen Verfahren kann durch die verwendete Konzentration der das Stickstoffmonoxid freisetzenden Agenzien und durch die physikalische und/oder chemische Induktion, welche für die Freisetzung von Stickstoffmonoxid aus den Agenzien verantwortlich ist, gesteuert werden. Gegebenenfalls kann der durch das erfindungsgemäße Verfahren erzeugte Stickstoffoxid enthaltende Trägergasstrom auf eine entsprechend medizinisch anzuwendende Konzentration noch verdünnt werden.

Dabei werden im Rahmen der vorliegenden Erfindung unter dem Begriff "physikalische und/oder chemische Induktion" neben der Intensität der elektromagnetischen Strahlung sowie der Expositionsdauer, welcher die Reaktionslösung ausgesetzt wird, im Allgemeinen auch die Reaktionsparameter verstanden, welche einen Einfluss auf die Bildung von Stickstoffmonoxid an sich und auf die Konzentration an Stickstoffmonoxid haben. Hierzu zählen im Allgemeinen der pH-Wert der Reaktionslösung, der Redoxstatus der Reaktionslösung, die Temperatur der Reaktionslösung, die exponierte Bestrahlungsfläche, die Zeit der Einwirkung einer Induktionsgrößen auf die Stickstoffmonoxid freisetzenden Agenzien, die Strömungsgeschwindigkeit des Trägergases, die Entfernung der Quelle der elektromagnetischen Strahlung zur Reaktionslösung, das Spektrum der elektromagnetischen Strahlungsquelle, die Absorptions-, Transmissions-, Reflexionseigenschaften der Reaktionslösung, die Konzentration von biologischen oder chemischen Katalysatoren oder Vermittlersubstanzen, die auch außerhalb der "typischen" physiko-chemischen Bedingungen einer optimalen NO-Freisetzung eine solche aus NO-generierenden Substanzen durch Katalyse oder entsprechende Akzeptoreigenschaften dennoch ermöglichen. Insbesondere sind damit Chromophore und andere Substanzen gemeint, mit deren Hilfe z.B. auch elektromagnetische Strahlung außerhalb des UVA-Spektrumsbereiches in der Lage sein könnte aus den entsprechenden NO-bildenden Agenzien eine NO-Freisetzung zu ermöglichen.

So ist es beispielsweise bei konstant gehaltenen Induktionsgrößen durch den Einsatz variierender Konzentrationen der Stickstoffoxid freisetzenden Substanz(en) möglich, variierende Mengen an Stickstoffmonoxid freizusetzen.

Ferner ist es bei einer konstanten Konzentration der Stickstoffoxid freisetzenden Substanz(en) möglich, die Freisetzung von Stickstoffmonoxid durch Variation der Einstellparameter der jeweiligen Induktionsgrößen zu verändern. Bei einer konstant gehaltenen Induktionsgröße können daher in dem erfindungsgemäßen Verfahren durch den Einsatz hoher Konzentrationen der NO-freisetzenden Substanzen hohe NO-Mengen freigesetzt werden und umgekehrt. Bei einer konstanten Konzentration der NO-freisetzenden Substanz kann die NO-Generierung durch Variation der Einstellparameter der jeweiligen Induktionsgrößen verändert werden. Die Einstellparameter können dabei alternativ oder gleichzeitig zur Regulation der NO-Erzeugung herangezogen werden. Insbesondere über die gleichzeitige Regelung der NO-Erzeugung über mehrere Einstellparameter kann das Verfahren vorteilhafterweise hinsichtlich der NO-Erzeugung sowie der Erzeugung unerwünschter Nebenprodukte optimiert werden.

Im Allgemeinen wird in dem erfindungsgemäßen Verfahren die Konzentration an Stickstoffmonoxid und an dem Stickstoffdioxidradikal als entscheidende Messgrößen verwendet. Die Bestimmung der Messgrößen erfolgt dabei im Allgemeinen im rückverdünnten Betriebsgas, das als Endprodukt in dem erfindungsgemäßen Verfahren erhalten wird. Stimmen diese Messgrößen nicht mehr mit den vorgegebenen Werten überein, kann durch Veränderung der Induktionsbedingungen eine Korrektur des Verfahrens erfolgen.

Die Substanz, welche zur Freisetzung von Stickstoffmonoxid verwendet und in dem erfindungsgemäßen Verfahren verwendet wird, unterliegt grundsätzlich keiner Beschränkung, solange sie in der Lage ist, unter dem Einfluss von elektromagnetischer Strahlung Stickstoffmonoxid freizusetzen. Sie kann beispielsweise aus der Gruppe bestehend aus
(a) reinen Stoffen oder Stoffgemischen, welche unter dem Einfluss elektromagnetischer Strahlung Stickstoffmonoxid generieren;
(b) Stoffgemische, die zusätzlich zu den unter (a) genannten Stoffen oder Stoffgemischen Hilfssubstanzen enthalten, welche ausgewählt sind aus der Gruppe, bestehend aus Photoakzeptoren, Photoamplifier, Enzyme oder Katalysatoren, um spontan oder unter physikalischem oder chemischem Einfluss Stickstoffmonoxid generieren; und
(c) Stoffe oder Stoffgemische, die erst als Folge einer zuvor stattgefundenen chemischen Reaktion unter Zuhilfenahme der unter (a) genannten Stoffe und gegebenenfalls der unter (b) genannten Hilfssubstanzen unter dem Einfluss von elektromagnetischer Strahlung spontan oder unter physikalischem oder chemischem Einfluss Stickstoffmonoxid generieren,
ausgewählt werden.

Ferner können die unter (a) beschriebenen Substanzen zusätzlich durch Temperaturveränderungen und/oder Feuchtigkeitsveränderungen und/oder pH-Veränderungen ihrer Lösungen und/oder Veränderungen des Redoxstatus ihrer Lösungen Stickstoffmonoxid freisetzen.

In einer Ausführungsform der vorliegenden Erfindung erfolgt die Freisetzung ausgehend von wässrigen Nitritlösungen. Dabei ist aus praktischen Gründen die Verwendung einer wässrigen Lösung von Natriumnitrit als Nitritquelle bevorzugt. Die wässrige Nitritlösung, insbesondere die wässrige Natriumnitritlösung, kann eine Konzentration an Nitrit von vorzugsweise 0,1 bis 10000 mM, insbesondere 0,2 bis 6000 mM, besonders bevorzugt 0,3 bis 5000 mM, speziell 0,4 bis 2000 mM, ganz speziell 0,5 bis 1500 mM, aufweisen.

Die Dauer der Herstellung von Stickstoffmonoxid durch das erfindungsgemäße Verfahren unterliegt keiner besonderen Beschränkung und hängt von dem Gesamtbedarf an Stickstoffmonoxid und der erforderlichen Konzentration an Stickstoffmonoxid ab. Aus praktischen Gründen wird das erfindungsgemäße Verfahren jedoch in der Regel für einen Zeitraum von vorzugsweise 1 Minute bis 24 Stunden, insbesondere 1 Sekunde bis 12 Stunden, besonders bevorzugt 1 Sekunde bis 6 Stunden, speziell 2 Sekunden bis 2 Stunden, durchgeführt.

Die Art der Bestrahlung des nitrithaltigen Ausgangssubstrats ist dem auf dem vorliegenden Gebiet tätigen Fachmann an sich bekannt. Es kann jedwede elektromagnetische Strahlung verwendet werden, welche in der Lage ist, Nitrit unter Bildung von Stickstoffmonoxid zu zersetzten. Beispielsweise kann im Rahmen der vorliegenden Erfindung die Herstellung von Stickstoffmonoxid mittels photolytischer Spaltung unter Verwendung einer UVA-Strahlung mit Wellenlängen von beispielsweise 320 bis 440 nm erfolgen. Es kann jedoch auch elektromagnetische Strahlung jeder anderer Wellenlänge verwendet werden, die allein oder unter Zuhilfenahme chemischer, physikalischer oder biologischer Verfahren eine photolytische Spaltung von Nitrit induziert.

Die erfindungsgemäße Herstellung von Stickstoffmonoxid kann unter einer Schutzgasatmosphäre erfolgen, wobei als Schutzgas beispielsweise Stickstoff oder Argon verwendet werden kann. Darüber hinaus sind auch alle weiteren Schutzgase im Rahmen der vorliegenden Erfindung verwendbar, solange sie sich gegenüber dem erfindungsgemäßen Reaktionssystem innert verhalten.

Das erfindungsgemäß erhaltene Stickstoffmonoxid wird vorzugsweise mit einem Trägermittel vermischt und in dieser erhaltenen Form als Betriebsgas den gewünschten Anwendungen zugeführt.

Als Trägermedium für das erfindungsgemäß freigesetzte Stickstoffmonoxid werden vorzugsweise ungiftige gasförmige Substanzen oder deren Gemische verwendet. Beispiele für das Trägergas sind Edelgase, Stickstoff (N₂), sauerstoffhaltige Gase, wie Luft oder definierte synthetische Gas- oder Luftgemische. Weitere Beispiele für das Trägermedium sind Flüssigkeiten (z.B. Stickstoffmonoxidgas gesättigte Kultur- oder Infusions-Medien bzw. -Puffer, Serum, Blut Gele) oder feste Stoffe.

In einer bevorzugten Ausführungsform des erfindungsgemäßen Verfahrens werden Vorkehrungen getroffen, welche eine erneute Bildung des Stickstoffdioxidradikals in dem Betriebsgas vermeiden. Hierzu zählt beispielsweise eine Kühlungsvorrichtung, durch welche das Betriebsgas geleitet wird und durch welche eine unerwünschte Folgereaktion in dem mit Stickstoffmonoxid angereicherten Betriebsgas unterdrückt werden kann. Durch die Kühlungsvorrichtung wird beispielsweise eine Rückreaktion zu dem Stickstoffdioxidradikal im Betriebsgas unterdrückt. Eine weitere Möglichkeit, eine unerwünschte Folgereaktion in dem mit Stickstoffmonoxid angereicherten Betriebsgas, beispielsweise eine Rückreaktion zu dem Stickstoffdioxidradikal, zu unterdrücken, besteht ferner darin, durch Absorptionsvorrichtungen oder Katalysatoren, insbesondere zur Reduktion des Stickstoffdioxidradikals, dessen Bildung im Betriebsgas im Wesentlichen oder vollständig zu unterdrücken.

Insgesamt ist in einer besonderen Ausführungsform der vorliegenden Erfindung das erfindungsgemäße Verfahren damit durch die folgenden Verfahrensschritte gekennzeichnet:
(1) Einfüllen von mindestens einer Ausgangssubstanz, welche zur Freisetzung von Stickstoffmonoxid geeignet ist, und mindestens eines Systems, welches mehrfach oxidierte Stickoxide, Sauerstoffradikalanionen oder Hydroxylradikale abbaut oder neutralisiert, in eine Reaktionskammer;
(2) lichtinduzierter Zerfall der Ausgangssubstanz durch Bestrahlung der Mischung aus Ausgangssubstanz und dem mindestens einen System unter Freisetzung von Stickstoffmonoxid;
(3) Entfernen des in Schritt (2) gebildeten Stickstoffmonoxids aus der Reaktionskammer mittels eines Trägermediums unter Erhalt eines Betriebgases, welches Stickstoffmonoxid enthält.

Das durch das erfindungsgemäße Verfahren hergestellte Stickstoffmonoxid kann beispielsweise zu Inhalationszwecken eingesetzt werden. Weitere spezifische Anwendungsgebiete sind die Anregung des Stoffwechsels von Geweben durch äußerliche Anwendung, die strukturelle Modifikation organischer sowie anorganischer Flächen, die Sterilisation oder die Erzeugung von Zytotoxizität. Das durch das erfindungsgemäße Verfahren erzeugte Stickstoffmonoxid kann auch zur Begasung von Wunden, insbesondere zur Wundheilung, der Oberhaut, insbesondere zum Peeling, von Warzen und von Tumoren, insbesondere im Rahmen von nicht invasiven Maßnahmen, eingesetzt werden. Das Stickstoffmonoxid kann, wenn es in gesättigten Flüssigkeiten erzeugt wird, systemisch zur Behandlung von Bluthochdruck eingesetzt werden. Schließlich kann das Stickstoffmonoxid auch in mit Stickstoffmonoxid nitrosierte Träger erzeugt werden, welche spontan wieder NO freisetzen und z.B. atopisch im Rahmen dermatologischer Therapien eingesetzt werden. Das Stickstoffmonoxid kann auch zur Herstellung unterschiedlicher NO-bindender Substanzen (z.B. NO-Donoren) genutzt werden.

Das vorliegende erfindungsgemäße Verfahren weist eine Vielzahl an Vorteilen auf:
Die Qualität eines gelagerten Gases für medizinische Anwendungen muss hohen Anforderungen genügen, die den Aufwand für Herstellung und Lagerung weiter erhöhen. Schon eine geringe Verunreinigung des Gases führt nämlich zur Bildung unerwünschter und eventuell giftiger Nebenprodukte. Die Entstehung dieser Nebenprodukte bei längerer Lagerung von Stickstoffmonoxid enthaltenden Gasflaschen sowie bei der plasmatechnischen Herstellung von Stickstoffmonoxid und die Entfernung dieser Radikale stellen einen großen technischen sowie finanziellen Nachteil dar. Die Vorteile des erfindungsgemäßen Verfahrens zur Herstellung von Stickstoffmonoxid-Gasgemischen sind die Einfachheit der Herstellungsmethode des NO-haltigen Gases, der besonders hohe Reinheitsgrad des hergestellten NO-Gasgemisches, geringe Folgekosten und keine Lagerungskosten, die besonders einfache Handhabung der NO-Erzeugung sowie der Reinheitskontrolle, das unvergleichlich günstige Verhältnis der Herstellungskosten zu der Menge des hergestellten NO-Gases.

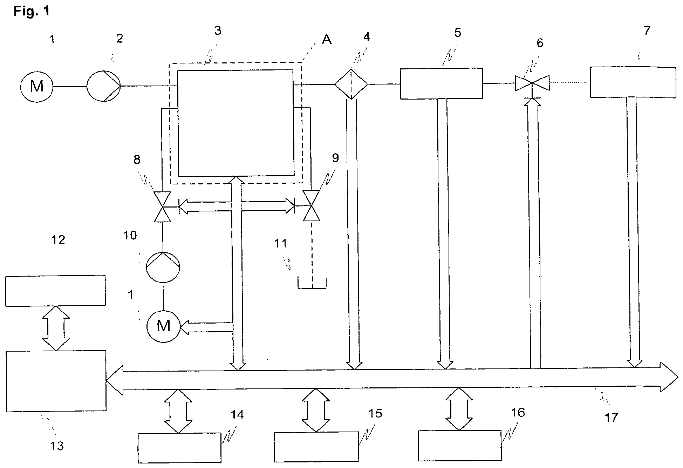

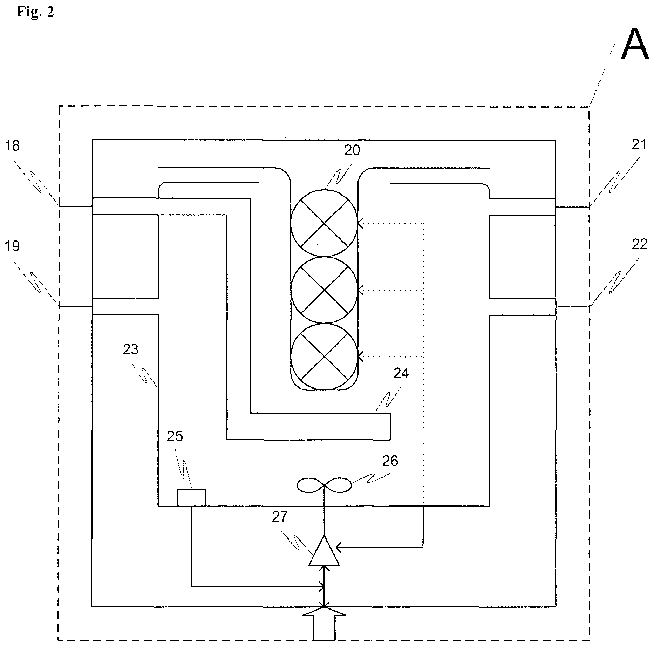

Im Folgenden wird eine zur Veranschaulichung eine Ausführungsform einer Vorrichtung zur Durchführung des erfindungsgemäßen Verfahrens anhand der Figuren 1 und 2 beschrieben, wobei die vorliegende Erfindung keinesfalls auf diese spezifische Ausführungsform beschränkt ist.

Fig. 1 zeigt schematisch den Aufbau einer Vorrichtung zur Herstellung von Stickstoffmonoxid. Durch die mit dem Bezugszeichen (1) gekennzeichneten Pumpenmotoren wird einerseits ein Trägergas oder Druckluft (2) in eine Reaktionskammer (3) geführt. Durch das Zuflussventil (8) ist es möglich, den Zufluss von NO-freisetzenden Substanzen zu der Reaktionskammer (3) zu variieren, d.h. zu begrenzen oder zu erhöhen. In der Reaktionskammer (3) findet die erfindungsgemäße Umsetzung unter Bildung von Stickstoffmonoxid statt. Aus der Reaktionskammer (3) kann durch das Abflussventil (9) Substrat entnommen werden. Das der Reaktionskammer (3) entnommene Stickstoffmonoxid-haltige Produkt wird anschließend einem Filter (4) für Stickstoffdioxid, einem Detektor (5) für Stickstoffmonoxid und für Stickstoffdioxid zugeführt. Der Gas-Strom, welcher der Reaktionskammer (3) entnommen und durch die Vorrichtungsbestandteile (21) geführt wird, wird anschließend über ein Sicherheitsventil (6) entnommen und seinem Ziel (7), beispielsweise einem Patienten, zugeführt. Durch den Detektor (5) können als Messgrößen die Konzentrationen an Stickstoffmonoxid und Stickstoffdioxid-Radikal bestimmt werden, so dass gegebenenfalls auf die Verfahrensführung Einfluss genommen werden kann.

Die vollständige Vorrichtung wird mittels einer CPU und einem Adress-, Daten- und Steuerbus gesteuert.

Fig. 2 zeigt schematisch den Aufbau der Reaktionskammer (3; A). Durch die Einleitung (18) wird das Trägergas eingeleitet und durch die Zuführung (19) wird das Nitrit in die Reaktionskammer geführt. Die Zuführung (18) ist mit einer Fritte (24) versehen. Durch den Mischer (26) mit einer Spannungsversorgung (27) wird das Reaktionsgemisch innig miteinander vermischt und mit einer entsprechenden UV-A-Lampe (20) bestrahlt. Das resultierende Stickstoffmonoxid kann der Öffnung (21) entnommen werden. Durch die Messapparatur (25) kann der in der Reaktionskammer (3; A) herrschende Druck, die herrschende Temperatur, die verwendete Lichtwellenlänge und -intensität bestimmt werden. Die hierdurch erhaltenen Daten werden in die entsprechende CPU übertragen.

Legende zu den Fig. 1 und 2:
- 1: Pumpenmotor
- 2: Trägergas- oder Druckluftpumpe
- 3: Reaktionskammer
- 4: NO₂-Filter
- 5: NO-, NO₂-Detektor
- 6: Sicherheitsventil
- 7: Patient
- 8: Zuflussventil
- 9: Abflussventil
- 10: NO₂-Pumpe
- 11: Abfluss
- 12: Benutzerschnittstelle
- 13: CPU
- 14: Speicher
- 15: Display
- 16: Audio
- 17: Adress-, Daten- und Steuerbus
- 18: Trägergas- oder O₂-Zufluss
- 19: NO₂-Zufluss
- 20: Lampen (UV-A)
- 21: NO-Abfluss
- 22: NO₂-Abfluss
- 23: Reaktionskammer
- 24: Fritte
- 25: Messapparatur(Druck, Temperatur, Lichtwellenlänge und -intensität, Medium-Konzentration...)
- 26: Mischer
- 27: Energiequelle

Die Vorteile der hier beschriebenen Vorrichtung beziehen sich auf die vollkommen fehlenden Lagerungskosten größerer NO-Gas-Lagerbestände. Aufgrund des Modul-Charakters der unterschiedlichen Reaktionskammern kann eine vordefinierte, kontrollierte Herstellung in einem großen Konzentrationsintervall des NO erreicht werden. Je nach Spezifikation der Kammer kann das produzierte NO zur professionellen Anwendung oder zu einer durch den Laien durchführbaren Anwendung genutzt werden. Vor dem Vermischen der unterschiedlichen Substanzen einer Reaktionskammer besitzen die einzelnen Chemikalien eine sehr lange Lebensdauer. Sie verderben nicht oder nur sehr langsam. Eine bei Flaschenlagerung nicht zu unterschätzende Gefahr der Vergiftung durch ausströmendes NO-Gas oder NO₂-Radikalenstehung ist nahezu ausgeschlossen, da nur verhältnismäßig geringe NO-Konzentrationen produziert werden. Alle für die Herstellung des NO-Gasgemisches notwendigen Chemikalien, technischen Vorrichtungen und Steuerungseinheiten sind sehr preiswert und einfach zu handhaben.

## Patentansprüche

1. Verfahren zur Herstellung von Stickstoffmonoxid durch photolytische Spaltung einer Ausgangssubstanz, **dadurch gekennzeichnet, dass** die Spaltung der Ausgangssubstanz, welche unter dem Einfluss elektromagnetischer Strahlung Stickstoffmonoxid freisetzt, in der Gegenwart von mindestens einem System durchgeführt wird, welches mehrfach oxidierte Stickoxide, Sauerstoffradikalanionen oder Hydroxylradikale abbaut oder neutralisiert.

2. Verfahren nach Anspruch 1, **dadurch gekennzeichnet, dass** ein Stickstoffmonoxid-haltiges Gasgemisch hergestellt wird.

3. Verfahren nach Anspruch 1 oder 2, **dadurch gekennzeichnet, dass** das System, welches mehrfach oxidierte Stickoxide, Sauerstoffradikalanionen oder Hydroxylradikale abbaut oder neutralisiert, ausgewählt ist aus der Gruppe, bestehend aus Ascorbinsäure, Ascorbat, Vitamin E, Derivate von Vitamin E, Thiole, Radikalfänger, Sauerstoffspezies oder Stickstoffspezies abbauende Enzyme.

4. Verfahren nach Anspruch 1 bis 3, **dadurch gekennzeichnet, dass** die photolytische Spaltung ausgehend von wässrigen Nitritlösungen erfolgt.

5. Verfahren nach Anspruch 4, **dadurch gekennzeichnet, dass** die wässrige Nitritlösung eine Konzentration an Nitrit von 0,1 bis 10000 mM, insbesondere 0,2 bis 6000 mM, besonders bevorzugt 0,3 bis 5000 mM, speziell 0,4 bis 2000 mM, ganz speziell 0,5 bis 1500 mM, aufweist.

6. Verfahren nach einem der Ansprüche 1 bis 5, **dadurch gekennzeichnet, dass** ein Betriebsgasstrom erzeugt wird, welcher eine Konzentration an Stickstoffmonoxid von weniger 0,5 - 1000 ppm oder höher aufweist.

7. Verfahren nach einem der Ansprüche 1 bis 6, **dadurch gekennzeichnet, dass** die Herstellung von Stickstoffmonoxid mittels UVA-Strahlung mit Wellenlängen von 320 bis 440 nm oder unter dem Einfluss elektromagnetischer Strahlung jeder anderer Wellenlänge erfolgt, die allein oder mit Unterstützung chemischer, physikalischer oder biologischer Verfahren eine photolytische Spaltung von Nitrit induziert.

8. Verfahren nach einem der Ansprüche 1 bis 7, **dadurch gekennzeichnet, dass** die Herstellung von Stickstoffmonoxid unter Normalbedingungen oder unter einer Schutzgasatmosphäre erfolgt.

9. Verfahren nach einem der Ansprüche 1 bis 8, **dadurch gekennzeichnet, dass** das Verfahren vorzugsweise im pH-Intervall von 0 bis 12, insbesondere von 1 bis 10, besonders bevorzugt von 2 bis 7, speziell von 2 bis 6, ganz speziell von 3 bis 4 durchgeführt wird.

10. Verfahren nach einem der Ansprüche 1 bis 9, **gekennzeichnet durch** die folgenden Verfahrensschritte: (1) Einfüllen von mindestens einer Ausgangssubstanz, welche zur Freisetzung von Stickstoffmonoxid geeignet ist, und mindestens eines Systems, welches Sauerstoffspezies abbaut oder neutralisiert, in eine Reaktionskammer; (2) lichtinduzierter Zerfall der Ausgangssubstanz durch Bestrahlung der Mischung aus Ausgangssubstanz und dem mindestens einen System unter Freisetzung von Stickstoffmonoxid; (3) Entfernen des in Schritt (2) gebildeten Stickstoffmonoxids aus der Reaktionskammer mittels eines Trägermediums unter Erhalt eines Betriebgases, welches Stickstoffmonoxid enthält.

11. Verfahren nach Anspruch 10, **dadurch gekennzeichnet, dass** das Trägermedium ausgewählt ist aus der Gruppe, bestehend aus für das freigesetzte Stickstoffmonoxid ungiftige gasförmige Substanzen oder deren Gemische, Edelgase, Stickstoff, sauerstoffhaltige Gase, wie Luft oder definierte synthetische Luftgemische, Flüssigkeiten, Gele oder feste Stoffe.

12. Verfahren nach einem der Ansprüche 1 bis 11, **dadurch gekennzeichnet, dass** die Freisetzung von Stickstoffmonoxid durch die verwendete Konzentration der Stickstoffmonoxid freisetzenden Agenzien und durch physikalische und/oder chemische Induktion, welche für die Freisetzung von Stickstoffmonoxid aus den Agenzien verantwortlich ist, gesteuert wird.

13. Verfahren nach Anspruch 12, **dadurch gekennzeichnet, dass** als physikalische und/oder chemische Induktion der pH-Wert der Reaktionslösung, der Redoxstatus der Reaktionslösung, die Temperatur der Reaktionslösung, der die Reaktionslösung umgebener Druck, die Intensität der elektromagnetischen Strahlung und die Expositionsdauer, welcher die Reaktionslösung ausgesetzt wird, die exponierte Bestrahlungsfläche, die Zeit der Einwirkung einer Induktionsgrösse auf die Stickstoffmonoxid freisetzenden Agenzien, die Strömungsgeschwindigkeit des Trägergases, die Entfernung der Quelle der elektromagnetischen Strahlung zur Reaktionslösung, das Spektrum der elektromagnetischen Strahlungsquelle, die Absorptions-, Transmissions-, Reflexionseigenschaften der Reaktionslösung, die Konzentration von biologischen oder chemischen Katalysatoren oder Vermittlersubstanzen, die auch ausserhalb der "typischen" physiko-chemischen Bedingungen einer optimalen NO-Freisetzung eine solche aus NO-generierenden Substanzen durch Katalyse oder entsprechende Akzeptoreigenschaften dennoch ermöglichen.

14. Verfahren nach einem der Ansprüche 1 bis 13, **dadurch gekennzeichnet, dass** in dem Verfahren die Konzentration an Stickstoffmonoxid und an Stickstoffdioxidradikal als Messgrössen verwendet werden, welche im rückverdünnten Betriebsgas ermittelt werden, das als Endprodukt aus der Vorrichtung zur Herstellung von Stickstoffmonoxid ausgeleitet wird.

15. Verfahren nach einem der Ansprüche 1 bis 14, **dadurch gekennzeichnet, dass** für das Betriebsgas, welches das freigesetzte Stickstoffmonoxid enthält, Vorkehrungen getroffen werden, die eine Bildung des Stickstoffdioxidradikals vermeiden.

## Claims

1. A method for the production of nitric oxide by means of the photolytic cleavage of a precursor, **characterized in that** the cleavage of the precursor that releases nitric oxide under the influence of electromagnetic radiation is carried out in the presence of at least one system that degrades or neutralizes polyoxidized nitrogen oxides, oxygen radical anions or hydroxyl radicals.

2. The method according to claim 1, **characterized in that** a gas mixture containing nitric oxide is produced.

3. The method according to claim 1 or 2, **characterized in that** the system that degrades or neutralizes polyoxidized nitrogen oxides, oxygen radical anions or hydroxyl radicals is selected from the group consisting of ascorbic acid, ascorbate, vitamin E, derivatives of vitamin E, thiols, radical traps, enzymes that break down oxygen species or nitrogen species.

4. The method according to claim 1 to 3, **characterized in that** the photolytic cleavage takes place starting from aqueous nitrite solutions.

5. The method according to claim 4, **characterized in that** the aqueous nitrite solution has a concentration of nitrite of 0.1 mM to 10,000 mM, especially 0.2 mM to 6000 mM, particularly preferably 0.3 mM to 5000 mM, especially 0.4 mM to 2000 mM, very specially 0.5 mM to 1500 mM.

6. The method according to one of claims 1 to 5, **characterized in that** a processing gas flow is generated that has a concentration of nitric oxide of less than 0.5 ppm to 1000 ppm or more.

7. The method according to one of claims 1 to 6, **characterized in that** the production of nitric oxide is carried out by means of UVA radiation at wavelengths between 320 nm and 440 nm or else under the influence of electromagnetic radiation of any other wavelength which, either alone or with the assistance of chemical, physical or biological methods, induces a photolytic cleavage of nitrite.

8. The method according to one of claims 1 to 7, **characterized in that** the production of nitric oxide is carried out under normal conditions or in an inert gas atmosphere.

9. The method according to one of claims 1 to 8, **characterized in that** the method is preferably carried out within a pH range from 0 to 12, particularly from 1 to 10, particularly preferred from 2 to 7, especially from 2 to 6 and very especially from 3 to 4.

10. The method according to one of claims 1 to 9, **characterized by** the following method steps:
(1) at least one precursor that is suited for releasing nitric oxide and at least one system that degrades or neutralizes oxygen species are filled into a reaction chamber;
(2) the precursor undergoes light-induced degradation through irradiation of the mixture consisting of the precursor and of the at least one system, thereby releasing nitric oxide;
(3) the nitric oxide formed in step (2) is removed from the reaction chamber by means of a carrier medium, thereby yielding a processing gas that contains nitric oxide.

11. The method according to claim 10, **characterized in that** the carrier medium is selected from the group consisting of gaseous substances or their mixtures that are non-toxic to the released nitric oxide, and also consisting of noble gases, nitrogen, gases containing oxygen such as air or defined synthetic air mixtures, liquids, gels or solids.

12. The method according to one of claims 1 to 11, **characterized in that** the release of nitric oxide is controlled by means of the employed concentration of the agents that release nitric oxide as well as by means of the physical and/or chemical induction that is responsible for the release of nitric oxide from the agents.

13. The method according to claim 12, **characterized in that** a physical and/or chemical induction encompasses the pH value of the reaction solution, the redox status of the reaction solution, the temperature of the reaction solution, the pressure surrounding the reaction solution, the intensity of the electromagnetic radiation, and the duration of the exposure to which the reaction solution is subjected, the surface area exposed to radiation, the duration of action of an induction quantity on the agents that release nitric oxide, the flow rate of the carrier gas, the distance between the source of electromagnetic radiation and the reaction solution, the spectrum of the source of electromagnetic radiation, the absorption, transmission and reflection properties of the reaction solution, the concentration of biological or chemical catalysts or mediators which, even outside of the "typical" physical-chemical conditions needed for an optimal NO release, nevertheless allow NO to be released from NO-generating substances through catalysis or through appropriate acceptor properties.

14. The method according to one of claims 1 to 13, **characterized in that**, in the method, the concentration of nitric oxide and the concentration of nitrogen dioxide radical are used as measured variables that are ascertained in the rediluted processing gas that is discharged as the final product from the device for the production of nitric oxide.

15. The method according to one of claims 1 to 14, **characterized in that** precautions have to be taken for the processing gas containing the released nitric oxide in order to prevent the formation of the nitrogen dioxide radical.

## Revendications

1. Procédé de production de monoxyde d'azote par clivage photolytique d'une substance initiale, **caractérisé en ce que** le clivage de la substance initiale, qui dégage du monoxyde d'azote sous l'effet d'un rayonnement électromagnétique, est réalisé en présence d'au moins un système qui décompose ou neutralise des oxydes d'azote, anions radicalaires d'oxygène ou radicaux hydroxyles.

2. Procédé selon la revendication 1, **caractérisé en ce qu'**on réalise un mélange gazeux contenant du monoxyde d'azote.

3. Procédé selon la revendication 1 ou 2, **caractérisé en ce que** le système qui décompose ou neutralise des oxydes d'azote, anions radicalaires d'oxygène ou radicaux hydroxyles est choisi dans le groupe composé de l'acide ascorbique, de l'ascorbate, de la vitamine E, de dérivés de la vitamine E, de thiols, de capteurs de radicaux, d'enzymes décomposant une espèce oxygénée ou une espèce azotée.

4. Procédé selon la revendication 1 à 3, **caractérisé en ce que** le clivage photolytique a lieu à partir de solutions aqueuses de nitrite.

5. Procédé selon la revendication 4, **caractérisé en ce que** la solution aqueuse de nitrite présente une concentration de nitrite de 0, 1 à 10000 mM, plus particulièrement de 0,2 à 6000 mM, particulièrement préférentiellement de 0,3 à 5000 mM, spécialement de 0,4 à 2000 mM, très spécialement de 0,5 à 1500 mM.

6. Procédé selon l'une des revendications 1 à 5, **caractérisé en ce qu'**est généré un flux de gaz de service qui présente une concentration de monoxyde d'azote inférieure à 0,5 - 1000 ppm ou plus.

7. Procédé selon l'une des revendications 1 à 6, **caractérisé en ce que** la production de monoxyde d'azote a lieu au moyen de rayonnement UVA avec des longueurs d'onde de 320 à 440 nm ou sous l'effet d'un rayonnement électromagnétique de toute autre longueur d'onde qui induit, seul ou à l'aide d'un procédé chimique, physique ou biologique, un clivage photolytique de nitrite.

8. Procédé selon l'une des revendications 1 bis 7, **caractérisé en ce que** la production de monoxyde d'azote a lieu dans des conditions normales ou sous atmosphère protectrice.

9. Procédé selon l'une des revendications 1 à 8, **caractérisé en ce que** le procédé est exécuté dans un intervalle de pH de 0 à 12, plus particulièrement de 1 à 10, particulièrement préférentiellement de 2 à 7, spécialement de 2 à 6, très spécialement de 3 à 4.

10. Procédé selon l'une des revendications 1 à 9, **caractérisé par** les étapes de procédé suivantes : (1) introduction, dans une chambre de réaction, d'au moins une substance initiale appropriée pour dégager du monoxyde d'azote et d'au moins un système qui décompose ou neutralise une espèce oxygénée ; (2) décomposition, induite par la lumière, de la substance initiale par irradiation du mélange composé de la substance initiale et de l'au moins un système avec dégagement de monoxyde d'azote ; (3) évacuation du monoxyde d'azote formé à l'étape (2) et contenu dans la chambre de réaction au moyen d'un fluide porteur avec obtention d'un gaz de service qui contient du monoxyde d'azote.

11. Procédé selon la revendication 10, **caractérisé en ce que** le fluide porteur est choisi dans le groupe comprenant des substances gazeuses non nocifs pour le monoxyde d'azote dégagé ou leurs mélanges, des gaz rares, l'azote, des gaz inertes, des gaz contenant de l'oxygène, tels que l'air ou des mélanges d'air synthétiques définis, des liquides, des gels ou des substances solides.

12. Procédé selon l'une des revendications 1 à 11, **caractérisé en ce que** le dégagement de monoxyde d'azote est contrôlé par la concentration utilisée des agents dégageant du monoxyde d'azote et par induction physique et/ou chimique responsable du dégagement de monoxyde d'azote par les agents.

13. Procédé selon la revendication 12, **caractérisé en ce que** sont utilisés, pour l'induction physique et/ou chimique, le pH de la solution réactive, le statut redox de la solution réactive, la température de la solution réactive, la pression à laquelle est exposée la solution réactive, l'intensité du rayonnement électromagnétique, et la durée de l'exposition, à laquelle est exposée la solution réactive, la surface d'irradiation exposée, le temps d'effet d'une grandeur d'induction sur les agents dégageant du monoxyde d'azote, la vitesse d'écoulement du gaz porteur, l'éloignement de la source de rayonnement électromagnétique par rapport à la solution réactive, le spectre de la source de rayonnement électromagnétique les caractéristiques d'absorption, de transmission et de réflexion de la solution réactive, la concentration des catalyseurs ou médiateurs biologiques ou chimiques qui permettent, même en dehors des conditions physico-chimiques « typiques » d'un dégagement optimal d'oxyde d'azote, un tel dégagement à partir de substances générant de l'oxyde d'azote, et ce, par catalyse ou du fait de caractéristiques appropriées des accepteurs.

14. Procédé selon l'une des revendications 1 à 13, **caractérisé en ce que** sont utilisées, dans le procédé, en tant que grandeurs de mesure, la concentration de monoxyde d'azote et celle de radical de dioxyde d'azote qui sont déterminées dans le gaz de service reconstitué qui est évacué en tant que produit final du dispositif de production de monoxyde d'azote.

15. Procédé selon l'une des revendications 1 à 14, **caractérisé en ce que** sont prises, pour le gaz de service qui contient le monoxyde d'azote dégagé, des dispositions pour éviter une formation du radical de dioxyde d'azote.
